# EUROPÄISCHE PATENTSCHRIFT

(11) **EP 0 362 667 B1**
(45) Veröffentlichungstag und Bekanntmachung des Hinweises auf die Patenterteilung: **21.04.1993**
(21) Anmeldenummer: 89117713.1
(22) Anmeldetag: 26.09.1989
(51) Int. Cl.: C07C 209/84, C07B 63/00

(54) **Verfahren zur Gewinnung alkylierter aromatischer Amine**
Method of producing alkylated aromatic amines
Procédé pour l'extraction d'amines aromatiques alkoyliques

(30) Priorität: 07.10.1988 DE 3834196
(43) Veröffentlichungstag der Anmeldung: 11.04.1990
(73) Patentinhaber: BAYER AG, 51368 Leverkusen (DE)
(72) Erfinder: Klein, Alfons, D-4000 Düsseldorf 1 (DE); Fiege, Helmut, Dr., D-5090 Leverkusen 1 (DE)

(56) Entgegenhaltungen:
- EP-A- 0 069 065
- EP-A- 0 150 770
- US-A- 3 678 113

## Beschreibung

Die Erfindung betrifft ein Verfahren zur Gewinnung von alkylierten aromatischen Aminen durch die Aufarbeitung roher katalysatorhaltiger Alkylierungsgemische solcher Amine aus der Alkylierung mit Hilfe von Olefinen.

Es ist bekannt, aromatische Amine und Diamine mit Olefinen in Gegenwart katalytischer Mengen von Aluminium/Aluminiumchlorid zu alkylieren (Angew. Chemie 69 (1957), 124-131). Diese Reaktion wurde erweitert durch die Anwendung von Aluminium/Zink/Aluminiumchlorid (EP 0 150 770). Es ist weiter bekannt, diese Reaktion nur in Gegenwart von AlCl₃ durchzuführen (EP 0 260 651).

Die Aufarbeitung der rohen Alkylierungsgemische nach der Alkylierungsreaktion erfolgt in bekannter Weise durch Ausrühren mit überschüssiger, verdünnter wäßriger Natronlauge. Dadurch wird der in der organischen Phase gelöste Kontakt zersetzt und in die wäßrig-alkalische Schicht übergeführt. Durch Schichttrennung kann das rohe alkylierte Amin gewonnen und der weiteren Aufarbeitung, beispielsweise durch Destillation, zugeführt werden.

Vor allem aus ökologischen Gründen ist dieses bekannte Aufarbeitungsverfahren von Nachteil: Bedingt durch die technische Bedeutung dieser Aminalkylierung wird sie im großen Maßstab durchgeführt, so daß als Nebenprodukt große Mengen Abfallnatronlauge anfallen. Die Abfallnatronlauge enthält aminische Verunreinigungen, die in Folge schlechter Schichttrennung in emulgierter Form vorliegen. Es ist nicht möglich, diese verunreinigte Abfallnatronlauge ohne einen aufwendigen Reinigungsprozeß zu entsorgen.

Es wurde nun ein Verfahren zur Gewinnung alkylierter aromatischer Amine durch Aufarbeitung roher Gemische aus der Alkylierung aromatischer Amine mit Olefinen mit einem Gehalt an Metall- und/oder Metallhalogenid-Katalysator gefunden, das dadurch gekennzeichnet ist, daß man dem Alkylierungsgemisch eine anorganische Base in einer Menge, die mindestens äquivalent zur Menge des Katalysatorhalogenids ist, und Wasser in einer Menge, die mindestens ausreichend zur Hydrolyse des gesamten Katalysatormetalls ist, zusetzt, danach das nach der Hydrolyse im Reaktionsgemisch vorhandene Wasser destillativ entfernt, den festen Katalysatorrückstand abtrennt und das verbleibende katalysatorfreie Alkylierungsgemisch einer üblichen weiteren Aufarbeitung zuführt.

Für die Alkylierung einzusetzende aromatische Amine sind solche der Formel 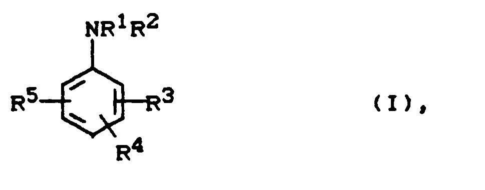
in der
- R¹ und R²: unabhängig voneinander Wasserstoff, Methyl oder durch R³, R⁴ und R⁵ substituiertes Phenyl bedeuten,
- R³ und R⁴: unabhängig voneinander Wasserstoff, geradkettiges oder verzweigtes C₁-C₁₀-Alkyl, geradkettiges oder verzweigtes C₁-C₁₀-Alkoxy, Phenyl, Fluor, Chlor oder Brom bedeuten und
- R⁵: für Wasserstoff, Methyl, Ethyl oder Amino steht.

In bevorzugter Weise werden für die Alkylierung aromatische Amine der Formel 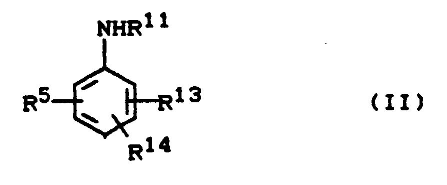
eingesetzt, in der
- R¹¹: Wasserstoff oder durch R⁵, R¹³ und R¹⁴ substituiertes Phenyl bedeutet,
- R¹³ und R¹⁴: unabhängig voneinander Wasserstoff, geradkettiges oder verzweigtes C₁-C₄-Alkyl, geradkettiges oder verzweigtes C₁-C₄-Alkoxy, Phenyl, Fluor oder Chlor bedeuten und
- R⁵: für Wasserstoff, Methyl, Ethyl oder Amino steht.

In besonders bevorzugter Weise werden für die Alkylierung aromatische Amine der Formel 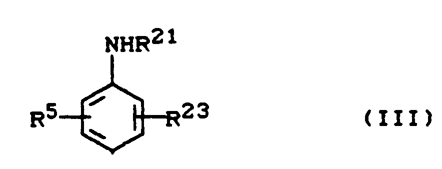
eingesetzt, in der
- R²¹: Wasserstoff, oder durch R⁵ und R²³ substituiertes Phenyl bedeutet,
- R⁵: für Wasserstoff, Methyl, Ethyl oder Amino steht und
- R²³: für Wasserstoff, geradkettiges oder verzweigtes C₁-C₄-Alkyl, geradkettiges oder verzweigtes C₁-C₄-Alkoxy oder Phenyl bedeutet.

Wichtige Beispiele für in der Alkylierung einsetzbare aromatische Amine enthält die folgende, keineswegs erschöpfende Aufzählung:
Anilin, o-, m- und p-Toluidin, die isomeren Xylidine, o-, m- und p-Ethylanilin, o-, m- und p-Iosopropylanilin, Diphenylamin, m-Phenylendiamin, Toluylendiamin-2,4, Toluylendiamin-2,6, 1-Isopropyl-phenylendiamin-2,4, 1-Isopropyl-phenylendiamin-2,6, 1-Ethyl-phenylendiamin-2,4, 1-Ethyl-phenylendiamin-2,6 u.a. Selbstverständlich können solche Amine auch im Gemisch mehrerer von ihnen zur Alkylierung eingesetzt und dann der erfindungsgemäßen Gewinnung zugeführt werden.

Die bei der Alkylierung einsetzbaren Alkene haben 2-10 C-Atome, bevorzugt 2-6 C-Atome, besonders bevorzugt 2-4 C-Atome und können geradkettig oder verzweigt sein. Ihre Doppelbindung kann endständig oder innenständig, bevorzugt endständig sein. Beispiele sind: Ethylen, Propylen, Buten-1, Buten-2, i-Buten, die isomeren Amylene, Hexene, Octene, Nonene oder Decene.

Als Katalysatoren für die Alkylierung werden solche metallhaltigen und/oder metallhalogenidhaltigen eingesetzt, die dem Fachmann für Friedel-Crafts-Alkylierungen bekannt sind. Insbesondere seien aluminiumhaltige Katalysatoren genannt. Die aluminiumhaltigen Katalysatoren können weitere Metalle wie Zink enthalten. Beispiele sind Aluminiummetall allein, Aluminiummetall/ AlCl₃, Aluminiummetall/Zinkmetall/AlCl₃ und AlCl₃ allein.

Bei einer solchen Alkylierung, die selbst nicht Gegenstand der vorliegenden Erfindung ist, werden die genannten aromatischen Amine im Kern ein- oder mehrfach alkyliert. Falls die Substituenten am N-Atom oder am Kern solcher aromatischen Amine Phenyl bedeuten, kann auch dieser Phenylsubstituent ein- oder mehrfach alkyliert werden. Die Art und der Umfang solcher Alkylierung ist unerheblich im Sinne der vorliegenden Erfindung. Von Bedeutung für die Erfindung und deren Ausgangspunkt ist vielmehr der Umstand, daß alle aus einer solchen Alkylierung stammenden Rohalkylate den verwendeten Katalysator enthalten, der zur Gewinnung der reinen alkylierten aromatischen Amine abgetrennt und entsorgt werden muß.

Als anorganische Basen für das erfindungsgemäße Verfahren können die Hydride, Oxide, Hydroxide, Carbonate, Hydrogencarbonate oder alkalisch reagierenden Salze anderer schwacher Säuren der Alkali- und Erdalkalimetalle eingesetzt werden. Andere schwache Säuren sind beispielsweise Borsäure, Essigsäure u.a. Bevorzugt werden die Hydroxide, Oxide oder Carbonate der Alkali- oder Erdalkalimetalle eingesetzt, besonders bevorzugt Natriumhydroxid, Kaliumhydroxid, Calciumhydroxid (gelöschter Kalk), Natriumcarbonat, Calciumcarbonat oder Calciumoxid (gebrannter Kalk), ganz besonders bevorzugt Natriumhydroxid, Calciumhydroxid oder Natriumcarbonat.

Die Menge der für erfindungsgemäße Verfahren einzusetzenden anorganischen Base kann in weiten Grenzen schwanken. Grundsätzlich steht sie im stöchiometrischen Zusammenhang mit dem eingesetzten Katalysator. Beispielsweise ist es möglich, so viel an Base einzusetzen, daß der Metallanteil und der Halogenanteil des Katalysators vollständig zu den entsprechenden, sich aus der anorganischen Base ergebenden Salzen reagieren. In einer besonderen Form ist es jedoch auch möglich, wesentlich weniger Base einzusetzen und zwar in einer Mindestmenge, daß die einzusetzende anorganische Base stöchiometrisch auf den Halogenanteil des Katalysators bezogen ist. Dies hat den Vorteil, daß eine geringere Menge Abfallsalz anfällt. Die einzusetzende Menge an anorganischer Base reicht also von der auf den Halogenanteil des Katalysators stöchiometrischen Menge bis zu einer aus wirtschaftlichen Gründen einzuhaltenden Obergrenze von 300 %, bevorzugt 150 % der auf den Metallanteil und den Halogenanteil des Katalysators bezogenen stöchiometrischen Menge dieser anorganischen Base.

Die Menge des für das erfindungsgemäße Verfahren einzusetzenden Wassers muß mindestens ausreichend zur Hydrolyse des gesamten Katalysatormetalls sein. Beim Einsatz von Alkali- oder Erdalkalihydroxiden als anorganische Base kann dieses mindestens einzusetzende Wasser auch das den Hydroxiden innewohnende Reaktionswasser sein, welches zu den Hydroxiden der Katalysatormetalle führt. Aus Gründen der besseren Reaktion der Katalysatorbestandteile mit der anorganischen Base ist es jedoch vorteilhaft, einen Überschuß einzusetzen. Dieser beträgt im allgemeinen das 1,1- bis 10-fache der theoretisch notwendigen Menge, bevorzugt das 1,5- bis 4-fache. Dieses Hydrolysewasser kann erfindungsgemäß in Form des Lösungs- oder Aufschlämmungswassers der anorganischen Base eingesetzt werden. Es ist aber auch möglich, Wasser und anorganische Base getrennt in beliebiger Reihenfolge einzusetzen.

Das im erfindungsgemäßen Verfahren durch Destillation wiedergewonnene überschüssige Wasser kann vorteilhaft als Teil des Hydrolysewassers eines Folgenansatzes eingesetzt werden. Dadurch arbeitet das erfindungsgemäße Verfahren in idealer Weise völlig abwasserfrei.

In einer weiteren vorteilhaften Variante wird das überschüssige Wasser durch azeotrope Destillation entfernt. Azeotropbildner für Wasser sind dem Fachmann bekannt; beispielsweise seien genannt: aromatische Kohlenwasserstoffe, wie Benzol, Toluol oder Xylol, (cyclo)aliphatische Kohlenwasserstoffe, wie Isooctan, Decan, Isododecan, Cyclohexan, Methylcyclohexan und andere. Sogar überschüssiges aus der Alkylierung stammendes Alken kann als Azeotropbildner benutzt werden, wenn es eine geeignete Siedelage hat; Beispiele hierfür sind Diisobutylen und Tripropylen. Alle Azeotropbildner werden in einer solchen Menge eingesetzt oder aus einem Wasserabscheider recyclisiert, daß alles überschüssige Wasser azeotrop abdestilliert werden kann; etwa vorhandenes Alken aus der Alkylierung, das als Azeotropbildner benutzt wird, fällt ebenso unter diese Mengenbestimmung.

Das erfindungsgemäße Verfahren wird bei 30-150°C, bevorzugt bei 50-140°C durchgeführt. Diese Temperatur ist weitgehend unkritisch. An die bei dieser Temperatur erfolgende Zersetzung des Katalysators schließt sich die Destillation des überschüssigen Wassers an, im Verlaufe derer die Katalysatorzersetzung vervollständigt wird. Die sich bei der Wasserdestillation einstellende Temperatur ist substratabhängig und ferner abhängig von der abnehmenden Konzentration des Wassers; sie kann bei Normaldruck oder bei Unterdruck, also im Bereich von 400-1000 mbar, ausgeführt werden. Solche Destillationen sind dem Fachmann geläufig.

Nach dem erfindungsgemäßen Verfahren wird das katalysatorhaltige Alkylierungsgemisch unter gutem Rühren mit der anorganischen Base und Wasser, bevorzugt in Form einer wäßrigen Lösung oder Aufschlämmung der anorganischen Base, versetzt. Dabei steigt die Temperatur, bedingt durch die exotherme Reaktion der Katalysatorzersetzung (Hydrolyse und Salzbildung, wie oben beschrieben) an. Nach Abklingen dieser exothermen Reaktion wird das Gemisch weiter erhitzt, wobei der zersetzte Katalysator ausfällt. Bei der bevorzugten Destillation unter Normaldruck beginnt ab 100°C Sumpftemperatur das Abdestillieren des Lösungs- und Reaktionswassers. Diese Wasserdestillation wird bis zu einer Sumpftemperatur von 140-160°C fortgeführt. Nach der Entfernung des Wassers liegt der zersetzte Katalysator im Alkylierungsgemisch als trockenes, körniges Salz vor. Die Abtrennung dieses Abfallsalzes kann in bekannter Weise durch Filtration, Abzentrifugieren oder Dekantieren erfolgen. Eine solche Abrennung, beispielsweise durch Filtration, kann bei Raumtemperatur erfolgen; vorteilhafter erfolgt sie jedoch bei erhöhter Temperatur von beispielsweise 30 bis 60°C. Das Filtrat ist frei von Salzen und Katalysatorrückständen sowie frei von Wasser und kann direkt einer üblichen weiteren Aufarbeitung, beispielsweise einer Feindestillation, zugeführt werden.

Das abgetrennte Abfallsalz (Katalysatorrückstand) kann durch Waschen mit einem organischen Lösungsmittel von kleinen Mengen des Alkylierungsgemisches befreit werden. Das hierzu benutzte organische Lösungsmittel richtet sich in einer dem Fachmann bekannten Weise nach der Löslichkeit der alkylierten Amine und kann beispielsweise ein aromatischer, aliphatischer oder cycloaliphatischer Kohlenwasserstoff sein, beispielsweise Benzol, Toluol, Xylol, Isooctan, Benzinfraktionen oder Cyclohexan. Das gewaschene Abfallsalz kann nach Entfernung des Waschmittels durch Trocknen oder Trockenblasen einer Deponie zur Entsorgung zugeführt werden. Es ist selbstverständlich, daß die aminhaltige Waschlösung und die beim Trocknen oder Trockenblasen freiwerdenden Lösungsmittel wiedergewonnen und geeigneten Kreisläufen zugesetzt werden.

### Beispiel 1

In das katalysatorhaltige Rohalkylat einer Ethylierung von 250 g eines Gemisches aus Toluylendiamin-2,4 und Toluylendiamin-2,6 im Verhältnis 65:35 mit 8,33 g Aluminiumchlorid und 4,72 g Aluminium/Zink 90:10 und Ethylen zu einem Gemisch aus 6-Methyl-2,4-diethylphenylendiamin-1,3 und 2-Methyl-4,6-diethylphenylendiamin-1,3 ließ man bei 40-50°C 39 g 20 %ige Natronlauge unter Rühren eintropfen. Die Temperatur stieg danach auf 80-90°C. Ein gelbliches Salz fiel aus. Man steigerte die Temperatur langsam bis 140°C und destillierte 21,8 g Wasser ab. Das salzhaltige Alkylat wurde zwischen 90 und 120°C abgesaugt. Auf der Nutsche verblieb das gelbliche Salz. Durch zweimaliges Waschen dieses Salzes mit je 25 ml Toluol ließ sich noch anhaftendes Alkylat herauslösen. Das auf diese Weise erhaltene Salz wog trocken 23,7 g.

### Beispiel 2

In das kontakthaltige Rohalkylat einer Ethylierung von 250 g Toluylendiamin-2,4 mit 8,33 g Aluminiumchlorid und 4,72 g Aluminium/Zink 90:10 und Ethylen zu 6-Methyl-2,4-diethylphenylendiamin-1,3 gab man bei 30-40°C 10,4 g Natriumcarbonat und 32 g Wasser und verfuhr wie in Beispiel 1 angegeben. Man erhielt 24,2 g Abfallsalz.

### Beispiel 3

In das kontakthaltige Rohalkylat einer Ethylierung von 290 g p-Toluidin mit 10,8 g Aluminiumchlorid und 5,4 g Aluminium und Ethylen zu 4-Methyl-2,6-diethyl-anilin gab man bei 40-50°C 45 ml Wasser und 7 g Calciumoxid und verfuhr wie in Beispiel 1 beschrieben. Man erhielt 29 g Abfallsalz.

### Beispiel 4

In das kontakthaltige Rohalkylat einer Propylierung von 240 g Anilin in Gegenwart von 40 g Aluminiumchlorid und 4 g Aluminium und Propylen zu 2,6-Diisopropyl-anilin ließ man bei 40 bis 50°C 185 g 20 %ige Natronlauge eintropfen und verfuhr wie in Beispiel 1 beschrieben. Es fielen 96 g Abfallsalz an.

### Beispiel 5

Das katalysatorhaltige Rohalkylat einer Umsetzung von 126,7 g Diphenylamin mit 266 g Diisobutylen in Gegenwart von 5 g Aluminiumchlorid zu 4,4′-Di(α,α,γ,γ-tetramethylbutyl)-diphenylamin wurde mit 13,3 g 45 %iger Natronlauge im Autoklav 2 Stunden bei 135°C gerührt. Anschließend wurde mit Hilfe des überschüssigen Diisobutylens das Wasser durch azeotrope Destillation entfernt. Dabei fielen 4,5 ml Wasser an. Nach der Filtration erhielt man 10,4 g Abfallsalz.

## Patentansprüche

1. Verfahren zur Gewinnung alkylierter aromatischer Amine durch Aufarbeitung roher Gemische aus der Alkylierung aromatischer Amine mit Olefinen mit einem Gehalt an Metall- und/oder Metallhalogenid-Katalystor, dadurch gekennzeichnet, daß man dem Alkylierungsgemisch eine anorganische Base in einer Menge, die mindestens äquivalent zur Menge des Katalysatorhalogenids ist, und Wasser in einer Menge, die mindestens ausreichend zur Hydrolyse des gesamten Katalysatormetalls ist, zusetzt, danach das nach der Hydrolyse im Reaktionsgemisch vorhandene Wasser destillativ entfernt, den festen Katalysatorrückstand abtrennt und das verbleibende katalysatorfreie Alkylierungsgemisch einer üblichen weiteren Aufarbeitung zuführt.

2. Verfahren nach Anspruch 1, dadurch gekennzeichnet, daß für die Alkylierung aromatische Amine der Formel 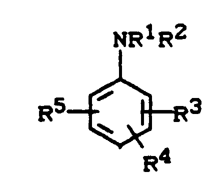 eingesetzt werden, in der
R¹ und R² unabhängig voneinander Wasserstoff, Methyl oder durch R³, R⁴ und R⁵ substituiertes Phenyl bedeuten,
R³ und R⁴ unabhängig voneinander Wasserstoff, geradkettiges oder verzweigtes C₁-C₁₀-Alkyl, geradkettiges oder verzweigtes C₁-C₁₀-Alkoxy, Phenyl, Fluor, Chlor oder Brom bedeuten und
R⁵ für Wasserstoff, Methyl, Ethyl oder Amino steht
bevorzugt für die Alkylierung aromatische Amine der Formel 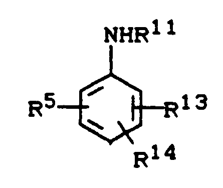 eingesetzt werden, in der
R¹¹ Wasserstoff oder durch R⁵, R¹³ und R¹⁴ substituiertes Phenyl bedeutet,
R¹³ und R¹⁴ unabhängig voneinander Wasserstoff, geradkettiges oder verzweigtes C₁-C₄-Alkyl, geradkettiges oder verzweigtes C₁-C₄-Alkoxy, Phenyl, Fluor oder Chlor bedeuten und
R⁵ für Wasserstoff, Methyl, Ethyl oder Amino steht
besonders bevorzugt für die Alkylierung aromatische Amine der Formel 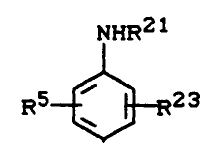 eingesetzt werden, in der
R²¹ Wasserstoff, oder durch R⁵ und R²³ substituiertes Phenyl bedeutet,
R⁵ für Wasserstoff, Methyl, Ethyl oder Amino steht und
R²³ für Wasserstoff, geradkettiges oder verzweigtes C₁-C₄-Alkyl, geradkettiges oder verzweigtes C₁-C₄-Alkoxy oder Phenyl bedeutet.

3. Verfahren nach Anspruch 1, dadurch gekennzeichnet, daß geradkettige oder verzweigte C₂-C₁₀-Olefine, bevorzugt C₂-C₆-Olefine, besonders bevorzugt C₂-C₄-Olefine eingesetzt werden.

4. Verfahren nach Anspruch 1, dadurch gekennzeichnet, daß aluminiumhaltige Katalysatoren, bevorzugt Al, Al/AlCl₃, Al/Zn/AlCl₃ oder AlCl₃, im rohen Alkylierungsgemisch vorliegen.

5. Verfahren nach Anspruch 1, dadurch gekennzeichnet, daß als anorganische Basen die Hydride, Oxide, Hydroxide, Carbonate, Hydrogencarbonate oder alkalisch reagierenden Salze anderer schwacher Säuren der Alkali- und Erdalkalimetalle eingesetzt werden.

6. Verfahren nach Anspruch 1, dadurch gekennzeichnet, daß die anorganische Base in einer Menge eingesetzt wird, die von der stöchiometrisch auf den Halogenanteil des Katalysators bezogenen bis zu 300 % der stöchiometrisch auf den Metallanteil und den Halogenanteil des Katalysators bezogenen, bevorzugt bis zu 150 % dieser Menge, reicht.

7. Verfahren nach Anspruch 1, dadurch gekennzeicnet, daß das Wasser in einer Menge zugesetzt wird, die das 1,1- bis 10fache, bevorzugt das 1,5- bis 4-fache, der zur Hydrolyse des Katalysatormetalls ausreichenden Menge beträgt.

8. Verfahren nach Anspruch 1, dadurch gekennzeichnet, daß das überschüssige Wasser bei 400-1000 mbar, bevorzugt unter Normaldruck bis zu einer Sumpftemperatur von 140-160°C destillativ entfernt wird.

9. Verfahren nach Anspruch 1, dadurch gekennzeichnet, daß zur destillativen Entfernung des überschüssigen Wassers ein Azeotropbildner zugesetzt wird.

10. Verfahren nach Anspruch 1, dadurch gekennzeichnet, daß das nach der Hydrolyse verbleibende und destillativ entfernte Wasser als Teil des Hydrolysewassers eines Folgeansatzes eingesetzt wird.

## Claims

1. Process for the isolation of alkylated aromatic amines by working up crude mixtures from the alkylation of aromatic amines with olefins containing metal and/or metal halide catalyst, characterised in that an inorganic base in an amount which is at least equivalent to the amount of the catalyst halide and water in an amount which is at least sufficient to hydrolyse all the catalyst metal are added to the alkylation mixture, the water present in the reaction mixture after the hydrolysis is then removed by distillation, the solid catalyst residue is separated off and the catalyst-free alkylation mixture which remains is fed to a customary further working up.

2. Process according to Claim 1, characterised in that aromatic amines of the formula 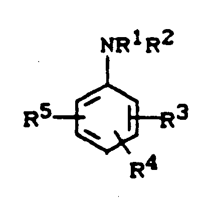 in which
R¹ and R² independently of one another denote hydrogen, methyl or phenyl which is substituted by R³, R⁴ and R⁵,
R³ and R⁴ independently of one another denote hydrogen, straight-chain or branched C₁-C₁₀-alkyl, straight chain or branched C₁-C₁₀-alkoxy, phenyl, fluorine, chlorine or bromine and
R⁵ represents hydrogen, methyl, ethyl or amino,
are employed for the alkylation, aromatic amines of the formula 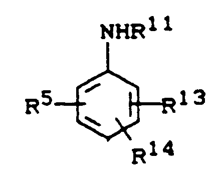 in which
R¹¹ denotes hydrogen or phenyl which is substituted by R⁵, R¹³ and R¹⁴,
R¹³ and R¹⁴ independently of one another denote hydrogen, straight-chain or branched C₁-C₄-alkyl, straight-chain or branched C₁-C₄-alkoxy, phenyl, fluorine or chlorine and
R⁵ represents hydrogen, methyl, ethyl or amino,
are preferably employed for the alkylation, and aromatic amines of the formula 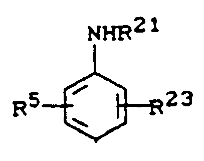 in which
R²¹ denotes hydrogen or phenyl which is substituted by R⁵ and R²³,
R⁵ represents hydrogen, methyl, ethyl or amino and
R²³ represents hydrogen, straight-chain or branched C₁-C₄-alkyl, straight-chain or branched C₁-C₄-alkoxy or phenyl,
are particularly preferably employed for the alkylation.

3. Process according to Claim 1, characterised in that straight-chain or branched C₂-C₁₀-olefins, preferably C₂-C₆-olefins and particularly preferably C₂-C₄-olefins, are employed.

4. Process according to Claim 1, characterised in that aluminium-containing catalysts, preferably Al, Al/AlCl₃, Al/Zn/AlCl₃ or AlCl₃ are present in the crude alkylation mixture.

5. Process according to Claim 1, characterised in that the hydrides, oxides, hydroxides, carbonates, bicarbonates or alkaline salts of other weak acids of the alkali metals and alkaline earth metals are employed as the inorganic bases.

6. Process according to Claim 1, characterised in that the inorganic base is employed in an amount which extends from the amount based stoichiometrically on the halogen content of the catalyst up to 300 % of the amount based stoichiometrically on the metal content and the halogen content of the catalyst, preferably up to 150 % of this amount.

7. Process according to Claim 1, characterised in that the water is added in an amount which is 1.1 to 10 times, preferably 1.5 to 4 times, the amount sufficient for hydrolysis of the catalyst metal.

8. Process according to Claim 1, characterised in that the excess water is removed by distillation under 400 to 1000 mbar, preferably under normal pressure, up to a bottom temperature of 140-160°C.

9. Process according to Claim 1, characterised in that an agent which forms an azeotrope is added for removal of the excess water by distillation.

10. Process according to Claim 1, characterised in that the water which remains after the hydrolysis and has been removed by distillation is employed as part of the hydrolysis water of a subsequent batch.

## Revendications

1. Procédé pour l'obtention d'amines aromatiques alkylées par traitement de mélanges bruts issus de l'alkylation d'amines aromatiques à l'aide d'oléfines, contenant un catalyseur métallique et/ou d'halogénure métallique, qui est caractérisé en ce qu'on ajoute, au mélange d'alkylation, une base inorganique en une quantité qui est au moins équivalente à la quantité de l'halogénure du catalyseur, ainsi que de l'eau en une quantité qui est au moins suffisante pour l'hydrolyse de l'ensemble du métal du catalyseur; ensuite, on élimine par distillation l'eau présente dans le mélange réactionnel après l'hydrolyse, on sépare le résidu solide de catalyseur et on achemine le mélange d'alkylation subsistant exempt de catalyseur à un traitement ultérieur habituel.

2. Procédé selon la revendication 1, caractérisé en ce qu'on met en oeuvre les amines aromatiques pour l'alkylation, répondant à la formule 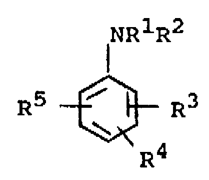 dans laquelle
R¹ et R² représentent, indépendamment l'un de l'autre, un atome d'hydrogène, un groupe méthyle ou un groupe phényle substitué par R³, R⁴ et R⁵,
R³ et R⁴ représentent, indépendamment l'un de l'autre, un atome d'hydrogène, un groupe alkyle en C₁-C₁₀ à chaîne droite ou ramifiée, un groupe alcoxy en C₁-C₁₀ à chaîne droite ou ramifiée, un groupe phényle, un atome de fluor, un atome de chlore ou un atome de brome, et
R⁵ représente un atome d'hydrogène, un groupe méthyle, un groupe éthyle ou un groupe amino,
de manière préférée, on met en oeuvre, pour l'alkylation, des amines aromatiques répondant à la formule 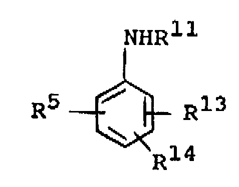 dans laquelle
R¹¹ représente un atome d'hydrogène ou un groupe phényle substitué par R⁵, R¹³ et R ¹⁴,
R¹³ et R¹⁴ représentent, indépendamment l'un de l'autre, un atome d'hydrogène, un groupe alkyle en C₁-C₄ à chaîne droite ou ramifiée, un groupe alcoxy en C₁-C₄ à chaîne droite ou ramifiée, un groupe phényle, un atome de fluor ou un atome de chlore, et
R⁵ représente un atome d'hydrogène, un groupe méthyle, un groupe éthyle ou un groupe amino,
de manière particulièrement préférée, on met en oeuvre, pour l'alkylation, des amines aromatiques répondant à la formule 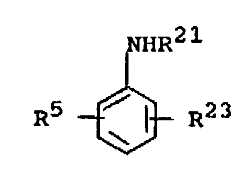 dans laquelle
R²¹ représente un atome d'hydrogène ou un groupe phényle substitué par R⁵ et R²³,
R⁵ représente un atome d'hydrogène, un groupe méthyle, un groupe éthyle ou un groupe amino et
R²³ représente un atome d'hydrogène, un groupe alkyle en C₁-C₄ à chaîne droite ou ramifiée, un groupe alcoxy en C₁-C₄ à chaîne droite ou ramifiée ou un groupe phényle.

3. Procédé selon la revendication 1, caractérisé en ce qu'on mot en oeuvre des oléfines en C₂-C₁₀ à chaîne droite ou ramifiée, de préférence des oléfines en C₂-C₆, de manière particulièrement préférée des oléfines en C₂-C₄.

4. Procédé selon la revendication 1, caractérisé en ce que des catalyseurs contenant de l'aluminium, de préférence Al, Al/AlCl₃, Al/Zn/AlCl₃ ou AlCl₃, sont présents dans le mélange brut d'alkylation.

5. Procédé selon la revendication 1, caractérisé en ce qu'on met en oeuvre, comme bases inorganiques, les hydrures, les oxydes, les hydroxydes, les carbonates, les hydrogénocarbonates ou les sels, à réaction alcaline d'autres acides faibles, des métaux alcalins ou alcalinoterreux.

6. Procédé selon la revendication 1, caractérisé en ce qu'on met en oeuvre la base inorganique en une quantité qui va d'une quantité rapportée stoechiométriquement à la fraction en halogène du catalyseur jusqu'à 300% de la quantité rapportée stoechiométriquement à la fraction métallique et à la fraction en halogène du catalyseur, de préférence jusqu'à 150% de cette quantité.

7. Procédé selon la revendication 1, caractérisé en ce qu'on ajoute l'eau en une quantité qui s'élève de 1,1 à 10 fois, de préférence de 1,5 à 4 fois la quantité suffisante pour l'hydrolyse du métal du catalyseur.

8. Procédé selon la revendication 1, caractérisé en ce qu'on élimine, par distillation, l'eau en excès sous une pression de 400 à 1.000 mbar, de préférence sous pression normale, jusqu'à une température de résidu de 140 à 160°C.

9. Procédé selon la revendication 1, caractérisé en ce que, pour l'élimination par distillation de l'eau en excès, on ajoute un agent formateur d'azéotrope.

10. Procédé selon la revendication 1, caractérisé en ce qu'on met en oeuvre l'eau subsistant après l'hydrolyse et éliminée par distillation, comme fraction de l'eau d'hydrolyse d'une charge ultérieure.
